# EUROPEAN PATENT APPLICATION

(11) **EP 1 632 205 A1**
(43) Date of publication of application: **08.03.2006**
(21) Application number: 05107591.9
(22) Date of filing: 18.08.2005
(51) Int. Cl.: A61F 9/007

(54) **Surgical apparatus**

(30) Priority: 02.09.2004 US 933916
(71) Applicant: Alcon, Inc., 6331 Hünenberg (CH)
(72) Inventor: Boukhny, Mikhail, Laguna Niguel, California 92677 (US)
(74) Representative: Hanna, Peter William Derek

(57) **Abstract**

A surgical handpiece for use in ophthalmic surgery, in particular cataract phacoemulsification surgery, is provided. A retention ring (110,210) that fits around a manipulator (112) or aspirating needle (212) protruding from the handpiece seals the distal end of a flexible irrigating sleeve (114,214) so as to reduce or prevent the flow of irrigation fluid out of the open distal end of sleeve and force the flow (120,220) of irrigating fluid out of the irrigating ports (116) on the side of the sleeve, and also helps prevent the collapse of the irrigating sleeve down around the manipulator or needle during insertion into the wound.

## Description

### Background of the Invention

This invention relates generally to the field of cataract surgery and more particularly to an apparatus for cataract phacoemulsification surgery.

The human eye in its simplest terms functions to provide vision by transmitting light through a clear outer portion called the cornea, and focusing the image by way of the lens onto the retina. The quality of the focused image depends on many factors including the size and shape of the eye, and the transparency of the cornea and lens.

When age or disease causes the lens to become less transparent, vision deteriorates lo because of the diminished light that can be transmitted to the retina. This deficiency in the lens of the eye is medically known as a cataract. An accepted treatment for this condition is surgical removal of the lens and replacement of the lens function by an artificial intraocular lens (IOL).

In the United States, the majority of cataractous lenses are removed by a surgical is technique called phacoemulsification. A typical surgical handpiece suitable for phacoemulsification procedures consists of an ultrasonically driven handpiece, an attached cutting tip, and irrigating sleeve and an electronic control console. The handpiece assembly is attached to the control console by an electric cable and flexible tubings. Through the electric cable, the console varies the power level transmitted by the handpiece to the attached cutting tip and the flexible tubings supply irrigation fluid to and draw aspiration fluid from the eye through the handpiece assembly.

The operative part of the handpiece is a centrally located, hollow resonating bar or horn directly attached to a set of piezoelectric crystals. The crystals supply the required ultrasonic vibration needed to drive both the horn and the attached cutting tip during phacoemulsification and are controlled by the console. The crystal/horn assembly is suspended within the hollow body or shell of the handpiece by flexible mountings. The handpiece body terminates in a reduced diameter portion or nosecone at the body's distal end. The nosecone is externally threaded to accept the irrigation sleeve. Likewise, the horn bore is internally threaded at its distal end to receive the external threads of the cutting tip. The irrigation sleeve also has an internally threaded bore that is screwed onto the external threads of the nosecone. The cutting tip is adjusted so that the tip projects only a predetermined amount past the open end of the irrigating sleeve.

A modified phacoemulsification technique called "bi-manual" phacoemulsification has been adopted by many surgeons. With the bi- manual technique, the irrigation sleeve is removed from around the ultrasonically driven tip. This allows for the small tip to be inserted into the eye through a smaller incision. Irrigation fluid is supplied by a second irrigating tip. The second tip may include a manipulation tool. Additional information concerning traditional phacoemulsification and bimanual phacoemulsification is included in U.S. Patent Publication No. US 2003/0069594 A1. As described in this reference, the second instrument does not use an outer silicone infusion sleeve. Rather the shaft of the tip is hollow with irrigation ports. Traditional phacoemulsification tips/irrigation sleeves have also been described as causing "roiling" in the area immediately in front of the phaco tip, and visibility reducing "clouding" of debris. According to this is reference, a bi-manual technique solves these and other problems. In the bi-manual technique, the incision may be smaller because the irrigation sleeve on the ultrasonic tip is not used, but without the sleeve, there is direct contact between the vibrating tip and the tissue at the wound. This can result in extra stress on the wound tissue, delaying healing and possibly requiring the use of a suture to seal the wound at the completion of surgery. The soft irrigation sleeve also acts to seal the wound from leakage during surgery. Without the use of the irrigation sleeve, excessive wound leakage can cause shallowing of the anterior chamber, excessive turbulence and premature removal of the protective viscoelastic material. Excessive wound leakage can also cause over-hydration of the wound tissue, possibly resulting in edema.

Recently, it has been suggested that traditional one-handed phacoemulsification can be conducted through a relatively small incision by reducing the diameter of the phacoemulsification tip/sleeve. A second irrigation/aspiration (I/A) tip, with or without an attached manipulation tool, may also be used to provide additional irrigation. Such an arrangement minimizes wound leakage, thereby helping to avoid over hydration of the wound, low intraocular pressure, excessive turbulence and premature removal of the viscoelastic material. The annular gap between the phaco tip and sleeve is used as an irrigation fluid pathway. Irrigation ports are provided on the sides of the sleeve to direct irrigating fluid out of and away from the aspiration port. This fluid flow out of the distal end of the sleeve tends to push material away from the aspiration port. In addition, the relatively unsupported distal end of the sleeve is compressed easily and pressed backward on the phaco tip or manipulator during insertion into the wound.

Traditional I/A tips are hollow needles having a closed end with an aspiration port and are used in the later part of the cataract extraction procedure to remove the remaining soft tissues of the lens. Minimizing the diameter of the I/A tip will increase the available amount of irrigation, but will also increase the amount of forward flowing fluid, resulting in the deficiencies discussed above.

Therefore, a need continues to exist for a device to seal and support the distal end of the irrigation sleeve on an irrigation/aspiration handpiece tip.

### Brief Summary of the Invention

The present invention provides surgical device having a retention ring that fits around a manipulator or aspirating needle and seals the distal end of a flexible irrigation sleeve so as to reduce or prevent the flow of irrigating fluid out of the open distal end of the sleeve and force the flow of irrigating fluid out of irrigating ports on the side of the sleeve and helps prevent the collapse of the irrigating sleeve down around the manipulator or needle during insertion into the wound. Accordingly, one objective of the present invention is to provide a device for reducing the flow of irrigating fluid out of the distal end of an irrigating sleeve. Another objective of the present invention is to provide a device for preventing the collapse of an irrigating sleeve on a surgical needle or manipulator during insertion into a wound. These and other advantages and objectives of the present invention will become apparent from the detailed description and claims that follow.

### Brief Description of the Drawings

FIG. 1 is a partial cross-sectional view of a prior art surgical manipulator having a flexible outer irrigating sleeve.
FIG. 2 is a partial cross-sectional view of a surgical manipulator having a flexible outer irrigating sleeve along with the seal of the present invention.
FIG. 3 is a partial cross-sectional view of a surgical needle having a flexible outer irrigating sleeve along with the seal of the present invention.

### Detailed Description of the Invention

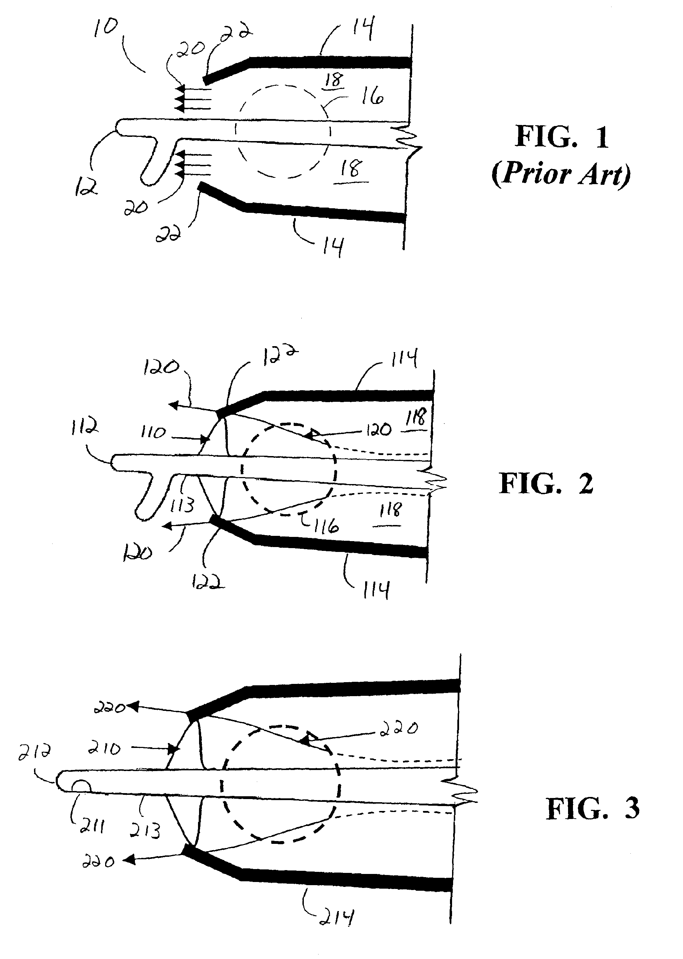

As best seen in FIG. 1, prior art devices 10 generally include relatively soft, flexible irrigation sleeve 14 coaxial about surgical manipulator 12. Manipulator 12 may be a hook, as shown in FIG. 2 or any other suitable manipulation device, such as a chopper, spatula or other desired device known in the art. Irrigation sleeve 14 contains ports or ports 16 that allow irrigating fluid flowing down gap 18 between manipulator 12 and sleeve 14 to exit out of the side of sleeve 14, but because manipulator 12 is generally made to be very small in diameter, gap 18 can be relatively large and allow flow 20 out of distal end 22 of sleeve 14. Unsupported distal end 22 of sleeve 14 may also be compressed, and collapse against manipulator 12 when entering a tight incision. Such is compression of sleeve 14 can cause sleeve 14 to be pulled backward on manipulator 12 during insertion into the wound.

As best seen in FIG. 2, the present invention generally includes a surgical manipulator 112 surrounded by relatively soft, flexible irrigation sleeve 114. Irrigation sleeve 114 contains ports or ports 116 that allow irrigating fluid flowing down gap 118 between manipulator 112 and sleeve 114 to exit out of the side of sleeve 114. Retention ring 110 is generally shaped like a ring or doughnut so as to be suitable for sliding onto shaft 113 of manipulator 112 and fit within distal end 122 of sleeve 114. Retention ring 110 may be made of any suitable material such as a moldable elastomer or thermoplastic, and sized and shaped to fit within commercially available sleeves 14. Alternatively, retention ring 110 may be integrally molded with sleeve 114. Retention ring 110 helps to prevent the flow of irrigating fluid out of distal end 122 of sleeve 114, instead tending to force or encourage any flow of irrigating fluid in gap 118 to flow out of port or ports 116, as shown by arrows 120. Retention ring 110 also helps prevent compression of sleeve 114 and collapse of sleeve 114 down about shaft 113.

Alternatively, as shown in FIG. 3, I/A tip 212 having shaft 213 and aspiration port 211 may be used with sleeve 214 and retention ring 210 to assist in creating irrigating flow 220 similar to that described with respect to FIG.2.

This description is given for purposes of illustration and explanation. It will be apparent to those skilled in the relevant art that changes and modifications may be made to the invention without departing from its scope.

## Claims

1. A surgical apparatus, comprising:
a) a shaft (113, 213) defining a tip adapted to function as a surgical tool (112, 212);
b) a relatively flexible irrigation sleeve (114, 214) having at least one port (116) and an open distal end (122), the irrigation sleeve generally coaxial about the shaft so as to form a gap (118) between the shaft and the irrigation sleeve, the gap being in fluid communication with the port and the open distal end;
**characterized by**
c) a retention ring (110, 210) sized and shaped to fit over the shaft and be held by the shaft in relation to the distal end of the sleeve, so as to reduce any irrigating fluid flow in the gap out of the distal end of the sleeve and to encourage any irrigating fluid flow (120, 220) in the gap out of the port, and/or so as to reduce any collapse of the sleeve down about the shaft.

2. The surgical apparatus of claim 1, wherein the surgical tool is a manipulator (112).

3. The surgical apparatus of claim 2, wherein the manipulator is a hook, chopper or spatula.

4. The surgical apparatus of claim 1, wherein the surgical tool is an aspiration needle (212).
